# EUROPEAN PATENT SPECIFICATION

(11) **EP 3 065 730 B1**
(45) Date of publication and mention of the grant of the patent: **25.12.2019**
(21) Application number: 14856949.4
(22) Date of filing: 04.11.2014
(51) Int. Cl.: A61K 31/198, A61K 36/534, A61K 36/67, A61P 9/08

(54) **VASODILATOR FORMULATION AND METHOD OF USE**
VASODILATORFORMULIERUNG UND VERFAHREN ZUR VERWENDUNG
FORMULATION DE VASODILATATEUR ET PROCÉDÉ D'UTILISATION

(30) Priority: 04.11.2013 AU 2013904256
(43) Date of publication of application: 14.09.2016
(73) Proprietor: Atp Institute Pty Ltd., Springwood QLD 4127 (AU)
(72) Inventor: LEGGE, Matthew, Loganholme, Queensland 4129 (AU)
(74) Representative: Schiweck Weinzierl Koch Patentanwälte Partnerschaft mbB
(86) International application number: PCT/AU2014/050333
(87) International publication number: WO 2015/061860

(56) References cited:
- EP-A1- 1 676 570
- WO-A2-2010/059963
- US-A1- 2003 130 183
- US-A1- 2005 069 597
- US-A1- 2007 196 301
- US-A1- 2010 227 925
- US-A1- 2011 070 315
- US-B1- 6 579 543
- I RUNNIE ET AL: "Vasorelaxation induced by common edible tropical plant extracts in isolated rat aorta and mesenteric vascular bed", JOURNAL OF ETHNOPHARMACOLOGY, vol. 92, no. 2-3, 1 June 2004 (2004-06-01) , pages 311-316, XP055365258, IE ISSN: 0378-8741, DOI: 10.1016/j.jep.2004.03.019

## Description

### TECHNICAL FIELD

THIS INVENTION relates to topical vasodilator formulation and its use. The formulations are useful for a variety of cosmetic and/or therapeutic applications such as for example enhancing muscular definition and to assist in the therapeutic treatment of diabetes.

### BACKGROUND

Approaches to improving blood flow to the skin have been many and consist of both systemic and topical approaches. Many beneficial effects can be experienced by a subject through an improvement in local blood flow, since impairment of local blood flow causes a variety of negative consequences. Among these are cold hands and feet, baldness, leg ulcers, certain forms of impotence, as well as a variety of other ailments.

Arginine is a naturally occurring amino acid, which participates in many important biochemical reactions essential to the normal physiology of a subject. This amino acid is found in most proteins consumed in a human's daily diet and can be metabolized to support glucose synthesis or catabolised to produce energy.

While many of the currently available topical vasodilator formulations increase or enhance blood flow to some degree, there is, nonetheless, a continued interest in identifying new formulations, which provide longer lasting vasodilator effects without undesirable side effects,

Accordingly, there is continued interest in the development of new topical vasodilator formulations.

### SUMMARY

The present invention is defined by the claims. The present disclosure comprising the invention is directed to formulations and their uses in methods of increasing or enhancing blood flow in a subject.

In a broad form, the invention relates to vasodilator formulations, for increasing, enhancing and/or stimulating blood flow to assist in the therapeutic treatment of diseases and/or conditions such as for example diabetes and in cosmetic applications such as for example the enhancement of muscle and vein definition in body builder, physique models, models, and/or aesthetically conscious individuals.

In a first aspect, a topically adnimistrable vasodilator formulation comprising:
(i) arginine,
(ii) black pepper extract, and
(iii) peppermint extract is provided.

In one embodiment, is selected from the group consisting of: 2-Amino-5-guanidinopentanoic acid, agmatine, arginine hydrochloride, Ark 1, decarboxylated arginine, dipeptide arginyl aspartate, D-arginine, L-arg, L-arginine, L-arginine aspartate, NG-monomethyl-L-arginine, arginine alpha ketogluterate, arginine-ethyl esther, norvaline, arginine salt, arginine ester, Argivene, Detoxargin, Levargin, Minophagen Argamine, Polyarginine, Arginina, (S)-2-Amino-5-guanidinopentanoic acid, 2-amino-5-guanidinovaleric acid, Argininum [INN-Latin], Arginina [INN-Spanish], L-alpha-Amino-delta-guanidinovaleric acid, L-Arginin, Poly(L-arginine), L-Ornithine, N5-(aminoiminomethyl)-, L(+)-Arginine, 1-Amino-4-guanidovaleric acid, H-Arg-OH, L-a-Amino-d-guanidinovaleric acid, L-Arginine, homopolymer, (S)-2-Amino-5-[(aminoiminomethyl)amino]pentanoic acid, L-Arginine hydrochloride, L-Norvaline, 5-((aminoiminomethyl)amino)-, (S)-2-Amino-5-guanidinovaleric acid, (2S)-2-amino-5-(diaminomethylideneamino)pentanoic acid, (S)-2-Amino-5-((aminoiminomethyl)amino)pentanoic acid, Pentanoic acid, 2-amino-5-((aminoiminomethyl)amino, L-Norvaline, 5-[(aminoiminomethyl)amino]-2-Amino-5-Guanidnovaleric Acid, Argininum Pentanoic acid, 2-amino-5-[(aminoiminomethyl)amino]-, Tocris-0663, L-Arginine (JP16), Lopac-A-5006, Arginine, 2-amino-5-guanidinovalerate, Arginine hydrochloride (USAN), L-a-Amino-d-guanidinovalerate, N5-(aminoiminomethyl)-L-Ornithine, L-alpha-Amino-delta-guanidinovalerate, (2S)-2-amino-5-guanidinopentanoic acid, 5-[(aminoiminomethyl)amino]-L-Norvaline, (2S)-2-amino-5-(carbamimidamido)pentanoic acid, L-Arginine-L-Glutamate 2-Amino-Pentanedoic Acid, (S)2-amino-5-[(aminoiminomethyl)amino]-Pentanoate, (S)-2-unino-5-[(aminoiminomethyl)amino]-Pentanoic acid, (2S)-2-azanyl-5-[bis(azanyl)methylideneamino]pentanoic acid, alpha-keto-gamma-guanidovaleric acid, ornithine, citrulline, guanidoacetic acid and ornithine.

Preferably, the vasodilator formulation comprises arginine hydrochloride.

In one embodiment the vasodilator formulation comprises from about 0.1% to 75% arginine. Preferably, the vasodilator formulation comprises about 2,50% arginine More preferably, the vasodilator formulation comprises about 2.5% arginine hydrochloride.

Suitably, the formulation comprises black pepper extract at a concentration of about 0.01 to 25%. Preferably, the formulation comprises black pepper extract at a concentration of about 0.25%.

In one embodiment, black pepper extract is black pepper oil.

Suitably, the formuLation comprises peppermint extract at a concentration of about 0.01 to 25%. Preferably, the formulation comprises peppermint extract at a concentration of about 0.25%.

In one embodiment, peppermint extract is peppermint oil.

In one embodiment, the topical formulation further comprises rosemary oil and/or one or more components or derivatives thereof. Preferably, the formulation comprises rosemary oil and/or one or more components or derivatives thereof, at a concentration of about 0.01 to 50% or preferably at a concentration of about 5-10%.

In one embodiment, the vasodilator formulation further comprises one or more penetration enhancers.

In one embodiment, the penetration enhancer is aloe vera extract or one or more components or derivatives thereof, and/or a fatty acid penetration enhancer. Preferably, the fatty acid penetration enhancer is stearic acid.

In one embodiment, the formulation comprises stearic acid at a concentration from about 1 to 75%. Preferably, the formulation comprises stearic acid at a concentration of about 5%.

Suitably, the formulation comprises aloe vera extract or one or mote components or derivatives thereof, at a concentration of about 0.01 to 20%. Preferably, the formulation comprises Aloe Barbadensis Leaf Juice or one or more components or derivatives thereof. Suitably, the Aloe Barbadensis Leaf Juice is present in the formulation at a concentration of about 2%.

In one embodiment, the Aloe Barbadensis Leaf Juice or one or more components or derivatives thereof, is peppermint oil

In one embodiment, the formulation may further comprise at least one terpene. Preferably, the terpene is from black pepper (*Piper nigrum*) extract or one or more components or derivatives thereof and/or peppermint extract or one or more components or derivatives thereof, More preferably, the terpene is from black pepper and/or peppermint oil

In one embodiment, the topical formulation further comprises one or more ecdysteroids.

In one embodiment, the topical formulation further comprises one or more antimicrobials.

The topical vasodilator formulation may further comprise one or more alpha-2 antagonists.

In one embodiment, the topical formulation further comprises one or more phosphodiesterase-5a inhibitors.

In one embodiment, the topical formulation further comprises lysine.

The topical vasodilator formulation may further comprise one or more alpha reductase inhibitors.

In one embodiment, the topical formulation further comprises one or more hair growth promoters.

In one embodiment, the topical formulation further comprises one or more immune and/or cytokine modulators. The topical vasodilator formulation may further comprise one or more additional vasodilators.

In one embodiment the additional vasodilators comprise nitric oxide based vasodilators. Suitably, the nitric oxide based vasodilators include for example Nitroglycerin, amyl nitrite, isobutyl nitrite, sodium nitroprusside, S-nitroso-N-acetylpenicillamine, isosorbide dinitrate, sildenafil, tetrahydrobiopterin, spironolactone, nitrites and nitrates.

In one embodiment, the topical formulation further comprises Hippophae rhamnoides extract or one or more components or derivatives thereof.

In one embodiment, the topical vasodilator formulation further comprises at least one pharmaceutically acceptable carrier, diluent and/or excipient. Preferably, at least one other pharmaceutically acceptable carrier, diluent and/or excipient may include one or more of a solubiliser, emollient, moisturiser, thickener, skin conditioner, preservative and/or stabiliser as would be understood by a person of skill in the art.

In one embodiment, the pharmaceutically acceptable carrier is an oil-in-water emulsion, Suitably, the oil-in-water emulsion is a low oil, high water emulsion.

In one embodiment, the pharmaceutically acceptable carrier, diluent and/or excipient further comprises an emulsifying wax. Preferably, the emulsifying wax is Cetearyl alcohol and/or Ceteareth-20.

In one embodiment the topical formulation is provided as an oil, mousse, gel, cream, lotion, foam, balm, ointment, liniment, liquid, aerosol, self-tanning lotion, oil, spray or paint and the like.

Preferably, the topical formulation is a cream or lotion.

In one embodiment, the present invention provides a formulation in the form of a vasodilator enhancer cream for topical application. The formulation comprises arginine, black pepper extract, peppermint extract, a skin penetration enhancer, aqua, natural emulsifying wax, ethyl-alcohol, ecdysterone, sorbitol, aloe barbadensis leaf juice, stearic acid, cyclopentasiloxane, tocopheryl, phenoxyethanol and caprylyl Glycol.

In a second aspect, the invention provides a method of producing the topically administrable vasodilator formulation of the first aspect, including the step of combining arginine, black pepper extract and peppermint extract to thereby produce the topically administrable vasodilator formulation.

The method of this aspect may further include combining one or more other ingredients or components of the formulation set forth in the first aspect.

In one embodiment, one or more of the components or ingredients of the formulation are micronized.

In one embodiment, the dry ingredient is at least one arginine or derivatives thereof, In one embodiment the dry ingredient is one or more ecdysteroids.

In a third aspect, the invention provides the claimed composition for use in a method of treating or preventing a disease, disorder or condition associated with a decrease and/or impairment of blood flow as defined in the claims in a subject, said method including the step of topically administering to said subject an effective amount of a topical vasodilator formulation according to the first aspect or produced according to the method of the second aspect, to increase, enhance and/or stimulate blood flow and/or vasodilation in the subject.

In one embodiment, the topical vasodilator formulation is topically administered to a subject at a location proximal to said decrease and/or impairment of blood flow.

In one instance, the disease, disorder or condition associated with a decreased or impaired blood flow and/or vasoconstriction is selected from the following non-limiting examples, including erectile dysfunction, aging, baldness, peripheral neuropathy, microangiopathy, female sexual dysfunction, male sexual dysfunction, diabetes, aging, restless leg syndrome, raynaud's phenomenon, Buerger's Disease, chilblains, numbness and tingling of extremities, varicose veins, haemorrhoids, hypothyroidism, immobility, cellulite, accumulation of subcutaneous adipose tissue, cosmetic applications such as poor quality hair, nail and skin, lymphedema, swelling of the hands and feet, oedema, deep vein thrombosis, ischemia, chronic venous insufficiency, gangrene, vasoconstriction, thrombosis, embolism, paresthesia, poikilothermia, cellulitis, tissue necrosis, ischaemic neuropathy, leg cramps either idiopathic, or related to either pregnancy, renal dialysis, or peripheral vascular disease (both venous and arterial), or to revitalize muscle, or to improve muscle strength and recovery after vigorous exercise and intense sport, or to improve muscle strength and performance during vigorous exercise and intense sport.

In one embodiment, the subject is a mammal. Preferably the subject is a human.

Alternatively, the subject is a non-human mammal, non-limiting examples of which include a horse, dog, cat, rabbit and the like.

In a fifth aspect, the invention provides a non-medical method of enhancing muscular and/or vascular definition in a subject, the method including the step of topically administering to said Subject an effective amount of a topical vasodilator formulation according to the first aspect, or produced according to the method of the second aspect, to enhance muscular and/or vascular definition in the subject

In one embodiment the subject is a body builder, physique model, model and/or an aesthetically conscious individual.

In a sixth aspect, the invention provides a kit comprising the topically administrable vasodilator formulation according to the first aspect, or produced according to the method of the second aspect, an applicator device and instructions for using said formulation to increase, enhance and/or stimulate blood flow and/or vasodilation in a subject.

In one embodiment, the applicator device is pump device.

### DETAILED DESCRIPTION

The present inventors have created an improved formulation which, when applied to the skin can effectively enhance blood flow and/or vasodilation when delivered to a target skin site.

Effective concentrations of the amino acid arginine and/or derivatives thereof, black pepper extract or one or more components or derivatives thereof, and peppermint extract or one or more components or derivatives thereof can be formulated to be topically applied.

The present invention provides a formulation as defined in the claims and its use in a method for prophylactically or therapeutically treating a disease, disorder or condition associated with impaired blood flow and/or vasoconstriction or in a non-medical method of enhancing muscular and/or vascular definition in a subject.

### Vasodilator Topical Farmulations

In a first aspect, the present disclosure comprising the invention provides a topically administrable vasodilator formulation comprising:
(i) arginine,
(ii) black pepper extract and
(iii) peppermint extract.

As used herein, the term *"extract"* refers to a composition or preparation comprising one or more active components, compounds or substances obtained, isolated or extracted from a particular source. The active components, compounds or substances in the extract may be in a more concentrated or enriched form compared to the source. In particular, the extract may be obtainable from a plant or any portion thereof, including for example peppermint.

The term *"derivative"* refers to a modified form of a particular compound or substance. The derivative may be a modified form of a compound or substance that is a component of the amino acid arginine for example. Typically, the derivative is a chemically modified or related form of the particular compound or substance.

As used herein, "% *concentration"* may refer to percent weight/volume (w/v), percent weight/weight (w/w) or percent volume/volume (v/v) of a particular ingredient within the formulation as applicable.

In one embodiment, the topical vasodilator formulation comprises from about0.1% to75% arginine.

Suitably, the formulation comprises at least one arginine at a concentration from about 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 1.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 2.1, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, 40, 40.5, 41, 41.5, 42, 42.5, 43, 43.5, 44, 44.5, 45, 45.5, 46, 46.5, 47, 47.5, 48, 48.5, 49, 49.5, 50, 50.5, 51, 51.5, 52, 52.5, 53, 53.5, 54, 54.5, 55, 55.5, 56, 56.5, 57, 57.5, 58, 58.5, 59, 59.5, 60, 60.5, 61, 61.5, 62, 62.5, 63, 63.5, 64, 64.5, 65, 65.5, 66, 66.5, 67, 67.5, 68, 68.5, 69, 69.5, 70, 70.5, 71, 71.5, 72, 72.5, 73, 73.5, 74, 74.5 to 75%.

Preferably, the vasodilator formulation comprises about 2.5% arginine.

In one embodiment, arginine is selected from the group consisting of: 2-Amino-5-guanidinopentanoic acid, agmatine, arginine hydrochloride, Ark 1, decarboxylated arginine, dipeptide arginyl aspartate D-arginine, L-arg, L-arginine, L-arginine aspartate, NG-monomethyl-L-arginine, arginine alpha ketogluterate, arginine-ethyl esther, norvaline, arginine salt, arginine ester, Argivene, Detoxargin, Levargin, Minophagen Argamine, Polyarginine, Arginina, (S)-2-Amino-5-guanidinopentanoic acid, 2-amino-5-guanidinovaleric acid, Argininum [INN-Latin], Arginina [INN-Spanish], L-alpha-Amino-delta-guanidinovaleric acid, L-Arginin, Poly(L-arginine), L-Omithine, N5-(aminoiminomethyl)-, L(+)-Arginine, 1-Amino-4-guanidovaleric acid, H-Arg-OH, L-a-Amino-d-guanidinovaleric acid, L-Arginine, homopolymer, (S)-2-Amino-5-[(aminoiminomethyl)amino]pentanoic acid, L-Arginine hydrochloride, L-Norvaline, 5-((aminoiminomethyl)amino)-, (S)-2-Amino-5-guanidinovaleric acid, (2S)-2-amino-5-(diaminomethylideneamino)pentanoic acid, (S)-2-Amino-5-((aminoiminomethyl)amino)pentanoic acid, Pentanoic acid, 2-amino-5-((aminoiminomethyl)amino, L-Norvaline, 5-[(aminoiminomethyl)amino]-2-Amino-5-Guanidnovaleric Acid, Argininum Pentanoic acid, 2-amino-5-[(aminoiminomethyl)amino]-, Tocris-0663, L-Arginine (JP16), Lopac-A-5006, Arginine, 2-amino-5-guanidinovalerate, Arginine hydrochloride (USAN), L-a-Amino-d-guanidinovalerate, N5-(aminoiminomethyl)-L-Ornithine, L-alpha-Amino-delta-guanidinovalerate, (2S)-2-amino-5-guanidinopentanoic acid, 5-[(aminoiminomethyl)amino]-L-Norvaline, (2S)-2-amino-5-(carbamimidamido)pentanoic acid, L-Arginine-L-Glutamate 2-Amino-Pentanedoic Acid, (S)-2-amino-5-[(aminoiminomethyl)amino]-Pentanoate, (S)-2-amino-5-[(aminoiminomethyl)amino]-Pentanoic acid, (2S)-2-azanyl-5-[bis(azanyl)methylideneamino]pentanoic acid, alpha-keto-gamma-guanidovaleric acid, ornitine, citrulline, guanidoacetic acid and ornithine.

Preferably, the formulation comprises arginine hydrochloride.

Suitably, the formulation comprises black pepper extract at a concentration of about 0.01 to 25%.

In one embodiment, the formulation comprises black pepper extract at a concentration from about 0.01,
0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5 to 25%.

Preferably, the formulation comprises black pepper extract at a concentration of about 0,25%,

In one embodiment, the black pepper extract is black pepper oil.

Suitably, the formulation comprises peppermint extract at a concentration of about 0.01 to 25%.

In one embodiment, the formulation comprises peppermint extract at a concentration from about 0.01,
0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5 to 25%.

In one embodiment, the peppermint extract
is peppermint oil.

In one embodiment, the topical formulation further comprises rosemary oil. As used herein *"rosemary"* may include species such as *Rosmarinus Officinalis* and *Rosmarinus coronarium,* although without limitation thereto. The oil may be an essential oil an infused oil or any other lipid-containing extract, fraction or infusion that comprises one or more therapeutically effective elements of rosemary.

Suitably, the rosemary oil is at a concentration ranging from about 0.01% to 50%, preferably about 0.05% to 25%, more preferably about 1% to 20%, even more preferably about 2% to 15%, or advantageously about 5-10%, inclusive of 6%, 7%, 8% and 9%.

Rosemary oil may improve the circulatory stimulant activity of the formulation disclosed herein.

The vasodilator formulation may further comprise one or more penetration enhancers to aid penetration of the active ingredients.

Suitably, penetration enhancers include without limitation: Oleic acid, 2 N-nonyl-1,3- dioxolanes, N-acetyle prolinate esters (such as pentyl- and octyl-N-acetyle prolinate), alkyldiloxanes (e.g., 1-Alkyl-3-β-D glucopyranosyl-1,1,3,3-tetramethyl disiloxanes), transcarbam (such as 5-(dodecyloxycarbonyl) pentylammonium-5- (dodecyloxycarbonyl) pentylcarbamate), iminosulfurane (like N-hexyl, N-benzoyl-S, S-dimethylimino-sulfuranes), capsaicin derivatives (e.g., Nonivamide), cinnamene compounds (such as cinnamic acid, cinnamaldehyde etc), terpenes, fatty alcohol, pyrrolidone, sulfoxides, laurocapram, surface active agents, amides, amines, lecithin, polyals, quaternary ammonium compounds, silicones, alkanoates and cardamom seed.

In one embodiment, the penetration enhancer is aloe vera extract or one or more components or derivatives thereof and/or a fatty acid penetration enhancer. Preferably, the fatty acid penetration enhancer is stearic acid.

Suitably, the formulation comprises aloe vera extract or one or more components or derivatives thereof at a concentration of about 0.01 to 20%. Preferably, the formulation comprises Aloe Barbadensis Leaf Juice or one or more components or derivatives thereof. Suitably, the Aloe Barbadensis Leaf Juice is present in the formulation at a concentration of about 2%.

In one embodiment, the Aloe Barbadensis Leaf Juice or one or more components or derivatives thereof is peppermint oil.

In one embodiment, the formulation comprises stearic acid at a concentration from about 1 to 75%. Preferably, the formulation comprises stearic acid at a concentration of about 5%.

Alternate fatty acid penetration enhancers include for example isostearic acid, lauric acid, myristic acid, capric acid, oleic, linoleic, linolenic acid, 1-carvyl esters, caproic acid/hexanoic acid, alkanoic acids, diacid, ethyloctadecanoic acid, lactic acid, linolaidic acid, neodecanoic acid, palmitic acid, pelargonic acid, propionic acid and vaccenic acid.

In one embodiment, the formulation may further comprise at least one terpene. Preferably, the terpene is from black pepper (*Piper nigrum*) extract or one or more components or derivatives thereof and/or peppermint extract or one or more components or derivatives thereof. More preferably, the terpene is from black pepper and/or peppermint essential oils.

In one embodiment, the topical vasodilator formulation further comprises an ingredient selected from the group consisting of: phytoecdysterones and ecdysones (e.g., ecdysteroids), antimicrobials, alpha-2 antagonists, phosphodiesterase-5a inhibitors, lysine, 5-alpha reductase inhibitor, hair growth promoters, immune and cytokine modulators, additional vasodilators and Hippophae rhamnoides extract or one or more components or derivatives thereof.

Ecdysteroids for inclusion in the formulation may be selected and/or sourced from the following non-limiting examples cyanatis vagas, Serratula, Silene species, Quinoa, chestnut and Leuzea, Suma extract, pfaffia extract, Brazilian ginseng extract, beta-ecdysterone, turkesterone, ecdysterone, Asparagus Filicinus, Spinacia oleracea, yams, white button Mushrooms, Ajuga Turkestanica, Rhaponticum carthamoides, Silene Praemixta, Vitex Scabra, as well as other Vitex species such as cymosa and canescens. Ecdysone, Ecdysterone, Beta-ecdysterone, 20-hydroxyecdysone, Turkesterone, Integristerone A, 24(28)-dehydramakisterone A, Viticosterone E, Sileneoside A and C, Ponasterone A, Cyasterone, 11-α-hydraxypoststerone, 9,11-Didehydropoststerone. Dacryhainansterone, 25-Hydroxydacryhainansterone, 14-Epi-20E, 24(23)-Dehydromakisterone A, 2-Deoxy-20E, 2-Deoxyecdysone, 2-Deoxyecdysone-22-acetate, Ajugasterone and Polypodine.

In one embodiment, the formulation comprises ecdysteroids at a concentration of about 5%. Suitably, the formulation may comprise the ecdysteroid 20-hydroxyecdysone at a concentration from about 1% to 50%. Preferably, the formulation may comprise 20-hydroxyecdysone at a concentration of about 5%.

Phytoecdysterones are plant based structural analogs of the insect moulting hormone ecdysone Phytoecdysteroids and ecdysones are commonly referred to as ecdysteroids. Ecdysteroids may serve as effective anabolic, hepatoprotective, immunoprotective, antioxidant and hypoglycemic agents. Ecdysteroids are general tonic and broad spectrum stimulants.

Antimicrobial ingredients for inclusion in the formulation may be selected from the following non-limiting examples, *Kunzea ambigua* extract or one or more components or derivatives thereof, eucalyptus extract or one or more components or derivatives thereof, Tea tree extract or one or more components or derivatives thereof, thyme extract or one or more components or derivatives thereof, Lavender extract or one or more components or derivatives thereof. Lemon extract or one or more components or derivatives thereof, Lemongrass extract or one or more components or derivatives thereof, Cinnamon extract or one or more components or derivatives thereof. Grapefruit extract or one or more components or derivatives thereof, Clove Bud extract or one or more components or derivatives thereof, Sandalwood extract or one or more components or derivatives thereof and Peppermint extract or one or more components or derivatives thereof.

In one embodiment, Lysine is added to the formulation. Preferably, the formulation further comprises Lysine at a concentration of about 0.5% to about 20%.

Alpha-2 antagonists for inclusion in the formulation may be selected from the following non-limiting examples. Yohimbine/rauwaoliscine, Aspidosperma quebracho-bianco extract or one or more components or derivatives thereof, Yohimbine Hcl, Yohimbe and pharmaceuticals atipamezole, efaroxan and idazoxan.

Phosphodiesterase-5a inhibitors for inclusion in the formulation may be selected from the following non-limiting examples: quercetin or its analogues thereof, icariin contained in Epimedium grandiflorum, 4-Methylpiperazine and Pyrazolo Pyrimidin-7-1, components of the lichen Xanthoparmelia scabrosa, red onion peel extract or one or more components or derivatives thereof, Eurycomanone and other quassinoids (commonly found in *Eurycoma longifolia*), or pharmaceuticals, Sildenafil citrate, tadalafil and vardenafil.

5-alpha reductase inhibitors for inclusion in the formulation may be selected from the following non-limiting examples: Saw Palmetto (serenoa repens) extract or one or more components or derivative thereof, nettle (urtica dioica) root extract or one or more components or derivatives thereof containing 3,4-divanillyltetrahydrofuran, hippophae rhamnoides, epilobium, Finasteride, Dutasteride and Alfatradiol.

In one embodiment, the formulation comprises 5-alpha reductase inhibitors at a concentration from about 0.5% to 25%. Preferably, the formulation comprises 5-alpha reductase inhibitors at a concentration of about 2.5%.

Hair growth promoters for inclusion in the formulation may be selected from the following non-limiting examples: *Eclipta alba, Crinum asiaticum* and L-ascorbate-2-phosphate.

Additional vasodilators for inclusion in the formulation may be selected from the following non-limiting examples: vitamin B3 niacinamide, nicotinic acid, methyl nicotinate, Sea Buckthorn (*Hippophae rhamnoides*) extract or one or more components or derivatives thereof and nitric oxide based vasodilators.

Suitably, the nitric oxide based vasodilators include for example Nitroglycerin, amyl nitrite, isobutyl nitrite, sodium nitroprusside, S-nitroso-N-acetylpenicillamine, isosorbide dinitrate, sildenafil, tetrahydrobiopterin, spironolactone, nitrites and nitrates.

Immune and/or cytokine modulators for inclusion in the formulation may be selected from the following non-limiting examples: Vitamin D, polypodium leucotomas, tocopherols and omega 3, 6, 7 and/or 9 oils.

Hippophae rhamnoides for inclusion in the formulation may be selected from the following non-limiting examples: Sea Buckthorn (Hippophae rhamnoides) extract or one or more components or derivatives thereof, in particular, Sea Buckthorn oil.

The topical formulation of the invention may further comprise a pharmaceutically acceptable carrier, diluent or excipient These include without limitation an effective amount of a moisturising, solubilising and/or substantive ingredient. A useful reference describing pharmaceutically acceptable carriers, diluents and excipients is Remington's Pharmaceutical Sciences (Mack Publishing Co NJ USA, 1991).

In one embodiment, the carrier is an oil-in-water emulsion. Suitably, the oil-tn-water emulsion is a low oil, high water emulsion. More suitably, the oil water ratio is from about 0.01 to 1 part oil to 1 part water. Preferably, the formulation is paraben free.

In addition to the above carrier, the formulation may also comprise Terpenes (Aqil, M et al., 2007, Drug Discovery Today, Vol 12, Issues 23-24, Pages 1061-1067; and Williams, A. C., and Barry, B. W., 1991, Pharmaceutical Research, vol. 8, Issue 1, Pages 17-24).

In one embodiment, terpenes include without limitation the following examples: Hemiterpenes, Monoterpenes, Sesquiterpenes, Diterpenes. Sesterterpenes, Triterpenes, Tetraterpenes, Polyterpenes. Alternatives may include Geraniol, Citronellol, Camphor. Pinenes (α and β) - Pine genera. Borneol, Rutaceae; Myrtaceae; Umbellifereae; Labiatae; Compositae; Pinaceae oils. Bergamot, Citronella, Laurel, Vetiver, Ginger, Sandalwood, Cinnamon, Nutmeg, cannabis sativa, Mentha × piperita, *Mentha canadenis, Mentha spicata* L. (M. viridis Linn.) and Mentha × cardiac, *Mentha arvensis,* oils are derived from *Eucalyptus polybractea, Eucalyptus smithii, Eucalyptus australiana* and *Eucalyptus globulus.*

Preferably, the terpenes in the formulation include black pepper extract or one or more components or derivatives thereof and menthol. More preferably, black pepper extract is black pepper oil. Suitably, black pepper extract is black pepper essential oil.

Suitably, black pepper essential oil comprises Limonene, Pinene, Myrcene, Phellandrene, Beta-caryophyllene, Beta-bisabolene, Sabinene, Linalol, Pinocarveol, Alpha Terpineol, Camphene and Alpha Terpenene.

In one embodiment, the formulation comprises black pepper oil at a concentration from about 0.01 to about 25%. Preferably, the black pepper oil is at a concentration of about 0.25%.

Terpenes are included in the list of Generally Recognized As Safe (GRAS) substances and have low irritancy potential. Their mechanism of percutaneous permeation enhancement involves increasing the solubility of drugs in skin lipids, disruption of lipid/protein organization and/or extraction of skin micro constituents that are responsible for maintenance of barrier status.

Terpenes are compounds naturally found in many essential oils including those from black pepper. Terpenes can enhance the permeation of both hydrophilic and lipophilic drugs. Black pepper essential oil induces a warming sensation when applied to the skin due to local dilation of microcirculation to the skin, which is capable of enhancing percutaneous absorption of the active ingredients.

In some embodiments, the vasodilator formulation has a semi-solid consistency of a mousse, gel, cream, lotion, self-tanning lotion, oil, spray or paint for ease of storage and application.

The vasodilator formulation preferably has the consistency of a cream.

In one embodiment, an effective amount of a thickener may be incorporated within the formulation to obtain the desired viscosity and consistency of the product for example, as use as a cream, lotion or ointment

Suitable thickening agents include acrylate polymers and co-polymers among the classes of polyacrylates, polymethacrylate, polymethylmethacrylates, polyacrylamides and their cross-linked derivatives, cellulose derivatives such as methyl cellulose, hydroxyethyl cellulose, hydroxypropylmethyl cellulose, and hydroxypropyl cellulose, sorbitol (glucitol), naturally derived gums such as xanthan gum, acacia gum, carob gum; alginate derivatives, pectin, carbomer, trolamine, glycerine and polysorbate.

The thickener is incorporated in an amount suitable to obtain the desired thickening effect.

Additionally, other ingredients such as but not limited to antioxidants, pH modifiers, perfumes, preservatives, and colours may be included within the formulation.

In one embodiment, the vasodilator formulation may be delivered using the following non limiting examples carbomer gel, serum, spray, bath oils, massage oils, patches, nanopatches, nanodelivery systems, compresses, poultices, tape, bandages, strapping tape, dose delivery devices, slow release devices, epidermal injection subcutaneous injection, dropper, clothing, dressings, gauze and/or injection.

### Method of production

In an aspect, the invention provides a method of producing the topical formulation disclosed herein. In one embodiment, the method includes the step of micronizing at least one of the dry ingredients. Preferably, the micronized ingredients are combined with a wetting agent or soluble carrier, before mixing with the remaining ingredients to produce the topical vasodilator formulation

In one embodiment, micronization of the dry ingredients is achieved through milling, bashing, macerating and/or grinding.

In one embodiment, the dry ingredient is at least one arginine or derivatives thereof. The dry ingredient may be one or more ecdysterones.

In one embodiment micronization of the dry ingredients is achieved through the following non-limiting examples: milling, bashing, maceration and/or grinding, mechanical impact mills (e.g., hammer mills and pin mills), fluid energy mills (e.g., spiral jet mills, pancake mills, loop jet mills or fluidized bed jet mills), RESS (Rapid Expansion of Supercritical Solutions) process, the SAS (Supercritical Anti-Solvent) method and the PGSS method (Particles from Gas Saturated Solutions) and Nanoparticalization.

The micronized dry ingredients may be combined with the remaining ingredients in any order to form the vasodilator formulation. Preferably, the micronized dry ingredients are combined with a wetting agent or soluble carrier, prior to being combined with the remaining ingredients. Optionally, the black pepper and/or peppermint extract or one or more components or derivatives thereof may be added last to the formulation

In a particular embodiment, the method includes heating stearic acid to form a liquid to which is added a vitamin E liquid. The mixed stearic acid and vitamin E liquid may be combined with the liquid phase (i.e water-soluble) ingredients. This may then be mixed in an emulsifying mixer. Emulsifying ointment may be added and mixing continued until lump free. This embodiment may further include the step of dissolving an ecdysteroid (e.g. micronized cyanotis extract) in ethanol. The cyanotis extract and ethanol may then be added to the emulsifying mixing drum and blended until smooth and of consistent color. The remaining essential oils and preservative ingredients are combined and then added to the emulsifying mixing drum and continue to blend until smooth and consistent This particular embodiment preserves the potency of the essential oils, Preserving the essential oil potency is assisted by avoiding the exposure to heat by only heating the stearic add to make it liquid. Combining other ingredients at room temperature will improve the efficacy of this formulation,

### Methods of Treatment and use of the Topical Formulation

The topical formulation of the disclosure comprising the invention finds use in increasing, enhancing and/or stimulating blood flow and/or vasodilation in a subject to assist in the therapeutic treatment of diseases and/or conditions such as for example diabetes and in cosmetic applications such as for example, the enhancement of muscle and vein definition in a body builder, physique model, model and/or an aesthetically conscious individual.

In a third aspect, the composition as defined in the claims for use in a method of treating or preventing a disease, disorder or condition associated with a decrease and/or impairment of blood flow and/or vasodilation in a subject is provided, said method including the step of topically administering to said subject an effective amount of a topical vasodilator formulation according to the first aspect or produced according to the method of the second aspect, to increase, enhance and/or stimulate blood flow and/or vasodilation in the subject.

In one embodiment, the topical vasodilator formulation is topically administered to a subject at the site of or at a location proximal to said decrease and/or impairment of blood flow and/or vasodilation.

The terms *"admnistration"* or *"administered"* describe the introduction of the topical vasodilator formulation, to a subject's skin.

The term *"therapeutically effective amount"* describes a quantity of the formulation of the first aspect or the formulation produced according to the method of the second aspect to achieve a desired effect in a subject being treated with the formulation For example, this can be the amount of the formulation necessary to increase, enhance and/or stimulate blood flow or vasodilation in a subject. In some embodiments, a "therapeutically effective amount" is sufficient to treat the vasoconstriction or decreased or impaired blood flow entirely. In other embodiments, a "therapeutically effective amount" is an amount sufficient to achieve an enhancement or increased stimulation of blood flow and/or vasodilation in a subject

Ideally, a therapeutically effective amount of the vasodilator formulation is an amount sufficient to induce the desired result without causing a substantial cytotoxic effect in the subject. The effective amount of the formulation of the first aspect or produced according to the method of the second aspect, useful for increasing, enhancing and/or stimulating blood flow in a subject will be dependent on the subject being treated, the type and severity of any associated disease, disorder and/or condition, and the manner of administration of the vasodilator formulation

By *"increasing", "enhancing"* or *"stimulating"* as in increasing, enhancing or stimulating vasodilation and blood flow in a subject, is meant a widening of the blood vessels and an increased amount of blood flowing through the blood vessels of a subject Such an increase need not restore the subject to their full health to be beneficial to the subject. Blood flow and vasodilation in a subject can be determined using any methods or standards known to the ordinarily skilled artisan, including both qualitative and quantitative methods and standards.

A *"prophylactic"* treatment is a treatment administered to a subject who does not exhibit signs of vasoconstriction or impaired blood flow and exhibits only early signs for the purpose of decreasing the risk of developing vasoconstriction.

In practicing the methods of the invention, the topical formulation may be administered to any topical site on a subject Topical sites of interest include without limitation: arms, legs, feet, hands; torso, head, etc. The surface area that is covered by the topical formulation following application must be sufficient to provide for the desired amount of formulation to provide vasodilating properties.

In one embodiment, the period of time that the topical vasodilator formulation is maintained at the site of application is about 48 hours. In a further embodiment, the time that the topical vasodilator formulation is maintained at the site of application is about 24 hours.

Suitably, the period of time during which the formulation is maintained at the application site is at least about 1, 2, 3, 4, 5, 10, 15, 20, 30, 45, 50 minutes, 1, 1.5, 2, 3, 3,5, 4, 5, 6, 7, 8, 9, 10, 12, 15, 20, 24, 48 or 72 hours. Suitably, the formulation is maintained at the site for as long as required by the subject to increase, enhance and/or stimulate vasodilation and blood flow.

In one embodiment, a given dosage of the topical vasodilation formulation may be applied as a single application or a plurality of applications over a given time period, e.g., for as long as the subject requires treatment, where the dosing schedule is administered over a given time period, examples of which include hourly, daily, weekly, biweekly or monthly dosing schedules.

In one embodiment, the formulation application site may be at multiple locations on a subject. The application site to which the topical formulation is applied will be sufficiently proximal to the body area on a subject that requires increased or enhanced blood flow, so that upon application of the formulation, the components of the formulation can readily reach the affected site and actively work to enhance vasodilation of the blood vessels and increase, enhance and/or stimulate blood flow.

Suitably, the vasodilator formulation is generally applied by the subject for a period of time sufficient for the desired amount of vasodilation to be achieved.

If a reduction in blood flow and/or vasodilation occurs following removal or non-use of the topical vasodilator formulation, further topical formulation may be applied. The process may be repeated as necessary and when desired by the subject to achieve effective vasodilation.

In some embodiments, the patient may experience vasodilation and/or enhanced blood flow either immediately or shortly after application. Suitably, the patient will experience at least some vasodilation effects about 1 to 60 seconds; 1, 2, 3, 4, 5, 6, 7, 8, 9, .10, 15, 20, 30, 40, 50, 60 minutes, or 1, 1.5, 2, 2.5, 3, 4, 5, 6, 7, 8, 9, 10, 15, 20, 25 hours or days following application of the topical formulation.

The amount of topical vasodilator formulation applied will usually be sufficient to cover the area of skin overlying the site of reduced blood flow so that the subject experiences vasodilation and an increase or enhanced blood flow. For solutions, liquids, gels, lotions, creams, ointments and the like, the topical formulation may be applied to the subject at a desired skin site and a covering optionally applied thereto. For example, such covering may include patches, bandages, plasters and dressings. An appropriate sized covering may be placed over the applied topical vasodilator formulation. Conveniently, the topical formulation may be provided in a unit dosage dispenser, such as for example a pump bottle, spray, dropper or roll-on device examples of which are well known in the art.

Upon application of the topical formulation, the components of the formulation penetrate the surface of the skin and the subject experiences vasodilation and increased blood flow. Suitably, the subject experiences at least a partial increase or enhancement of blood flow. Preferably, the subject experiences restored blood flow and in some cases may experience a complete restoration of blood flow. Accordingly, application of the topical formulation in accordance with the methods of the invention results in treatment of the subject suffering from vasoconstriction and/or impairment of blood flow.

The topical formulation of the invention is available to a plurality of subjects. The term "*subject"* is used in its broadest sense. In a preferred embodiment, the subject is a mammal. More preferably, the subject is a human. Non-limiting examples of mammals include humans, dogs, cats, horses, cows, sheep, goats and pigs. Preferably, a subject includes any human or non-human mammal, including for example a primate, cow, horse, pig, sheep, goat, dog, cat, or rodent.

By "*treatment"* is meant at least an amelioration of the vasoconstriction or reduced blood flow experienced by a subject, where amelioration is used in a broad sense to refer to at least a reduction in the magnitude of a parameter, e.g. vasoconstriction, associated with the condition, disorder or disease being treated. As such, treatment also includes situations where the vasodilation and/or blood flow is completely restored, e.g., such that the host no longer suffers from vasoconstriction or reduced blood flow. The beneficial effect of the vasodilator formulation can be determined using any methods or standards known to a person of skill in the art.

The topical vasodilator formulation of the disclosure comprising the invention may be used to treat vasoconstriction and/or reduced blood flow associated with many conditions by topically applying the formulation to the effected site as described in the aforementioned embodiments. Specifically, the topical formulation may be used to treat vasoconstriction and/or reduced blood flow, including, but not limited to: erectile dysfunction, peripheral neuropathy, microangiopathy, female sexual dysfunction, male sexual dysfunction, diabetes, aging, restless leg syndrome raynad's phenomenon, Buerger's Disease, chilblains, numbness and tingling of extremities, varicose veins, haemorrhoids" hypothyroidism, immobility, cellulitis accumulation of subcutaneous adipose tissue, cosmetic applications such as poor quality hair, nail and skin, lymphedema, swelling of the hands and feet, oedema, deep vein thrombosis, ischemia, chronic venous insufficiency, gangrene, vasoconstriction, thrombosis, embolism, paraesthesia, poikilothermia, cellulites, tissue necrosis, ischaemic neuropathy, leg cramps either idiopathic, or related to either pregnancy, renal dialysis, or peripheral vascular disease (both venous and arterial), or to revitalize muscle, or for a non-medical use to improve muscle strength and recovery after vigorous exercise and intense sport, or to improve muscle strength and performance during vigorous exercise and intense sport.

### Method of Enhancing Muscular and/or Vascular Definition

In a fifth aspect, the invention provides a non-medical use in a method of enhancing muscular and/or vascular definition in a subject, the method including the step of topically administering to said subject an effective amount of a topical vasodilator formulation according to the first aspect or produced according to the method of the second aspect, to enhance muscular and/or vascular definition in the subject

In one embodiment the subject is a body builder, physique model, model and/or aesthetically conscious individual.

In one embodiment, the vasodilator formulation may be applied to one or more target sites on a subject for a period of time to achieve a desired enhancement of muscular and/or vascular definition. Suitably, the vasodilator formulation may be applied to one or more target sites on a subject for up to 60 minutes prior to exercise, during exercise, or up to 60 minutes post exercise.

In one embodiment, for example, the vasodilator formulation may be applied to one or more target sites on a subject prior to modelling, physique and/or body building competitions or presentations.

In one embodiment the vasodilator formulation may be applied to one or more target sites on a subject that wishes to improve and/or enhance muscular and/or vascular definition.

### Kits

In a sixth aspect, the invention provides a kit comprising the topically administrate vasodilator formulation according to the first aspect or produced according to the method of the second aspect, an applicator device and instructions for using said formulation to increase, enhance and/or stimulate blood flow and/or-vasodilation in a subject.

The applicator device can be any device that ensures correct and targeted delivery of the vasodilator formulation of the first aspect or produced according to the method of the second aspect. Examples of appropriate devices include applicators that attach to a single or multi-dose container of the formulation, tubes, roll-on device, brush, sponges, spray, aerosol, syringe or droppers.

Preferably, the applicator device is a pump device.

The kit may also include instructions for how to use the formulation, where the instructions typically include information about how to apply the formulation, dosing schedules etc. The instructions are generally recorded on a suitable recording medium. For example, the instructions may be printed on a substrate, such as paper or plastic, etc. As such, the instructions may be present in the kits as a package insert, in the labelling of the container of the kit or components thereof (i.e, associated with the packaging or sub packaging) etc. In other embodiments, the instructions are present as an electronic storage data file.

Throughout this specification, the aim has been to describe the preferred embodiments of the invention without limiting the invention to any one embodiment or specific collection of features. Various changes and modifications may be made to the embodiments described and illustrated herein without departing from the scope of the claims.

### EXAMPLES

The topical vasodilator formulations may be provided as an oil, mousse, gels, creams, lotions, balms, foams, liquids, aerosols, self-tanning lotion, spray or paint and the like.

The following examples and accompanying tables provide embodiments of the vasodilator formulations of the invention, methods for preparing same and methods of administration to a subject.

### Example 1

A topical vasodilator formulation was prepared comprising 2. 5% Arginine Hcl 0.25% Black Pepper Essential oil and 0,25% Peppermint essential oil as per the following formulation (Table: 1) to provide a formulation Suitable to be used for example in a pump device.

The formulation may be used to enhance muscular and/or vascular definition in a subject, in particular a subject practicing body building.

Approximately, 5% *Cyanotis vaga* extract supplying 50% 20-Hydroxyeedysterone and 2.5% arginine Hcl was micronized to form a powder and combined with the wetting; agent ethanol. In a separate container, the base was formed through beating the oil phase in a container to 60 degrees and then heating the water phase in a separate container to 60 degrees. Both the oil and water phase were then combined to form an emulsion. The micronized powders 5% *Cyanotic vaga* extract supplying 56% 20-Hydroxyecdysterone and 2,5% arginine Hcl are added to the base using a geometric dilution. The black pepper and peppermint essential oils were further combined with the formulation in an ointment mill to form an active blend.

Optionally, the formulation may comprise alternative ecdysterones.

The formulation may then be transferred to a suitable application device such as for example a pump device, which allows for an easy, no mess application by a subject.

### Example 2

A topical vasodilator formulation was prepared comprising 2.5% Arginine Hcl, 0.25% Black Pepper Essential oil, 0,25% Peppermint essential oil as per the following formulation (Table 2) to provide a formulation suitable to be used for example in a pump device.

The formulation may be used in the treatment of a disease, disorder or condition associated with a decrease and/or impairment of blood flew in a subject, in particular targeting peripheral blood flow, suitably diabetes.

Approximately, 5% Cyanotis vaga extract supplying 50% 20-Hydroxyecdysterone and 2.5% arginine Hcl was micronized to form a powder and combined with the wetting agent ethanol. The base was formed through heating the oil phase in a container to 60 degrees and then heating the water phase in a separate container to 60 degrees. Both the oil and water phase were then combined to form an emulsion. The micronized powders of 5% Cyanotis vaga extract supplying 50% 20-Hydroxyecdysterone and 2.5% arginine Hcl to the base using a geometric dilution were combined. Black pepper and peppermint essential oil were combined with the mixture in an ointment mill to form an active blend.

optionally, the formulation may further comprise an antimicrobial antiseptic, such as for example. *Kunzea ambigua* extract or a component or derivative thereof and Tea tree oil extract or a component or derivative thereof or an alternative ecdysteroid may be added to the vasodilator formulation.

The formulation may then be transferred to a suitable application device such as for example a pump device, which allows for an easy, no mess application.

### Example 3

A topical vasodilator formulation was prepared comprising 2.5% Arginine Hcl, 0.25% Black Pepper Essential oil and 0.25% Peppermint essential oil as per the following formulation (Table 3) to provide a formulation suitable to be used for example in a pump device.

The formulation may be used in the treatment of a disease, disorder or condition associated with a decrease and/or impairment of blood flow in a subject, in particular to treat erectile dysfunction and female sexual dysfunction.

Approximately, 5% Cyanotis vaga extract supplying 50% 20-Hydroxyecdysterone and 2.5% arginine Hcl was micronized to form a powder and combined with the wetting agent ethanol. The base was formed through heating the oil phase in a container to 60 degrees and then beating the water phase in a separate container to 60 degrees. Both the oil and water phase were then combined to form an emulsion. The micronized powders of 5% Cyanotis vaga extract supplying 50% 20-Hydroxyecdysterone and 2.5% arginine Hcl to the base using a geometric dilution were combined. Black pepper and peppermint essential oil were combined with the mixture in an ointment mill to form an active blend.

Optionally, the formulation may further comprise a PDE inhibitor, Quercetin or derivatives thereof alpha-2 adrenoceptor antagonist yohimbine Hcl or derivatives thereof, Lysine or a lubricant or an ingredient such as glow in the dark compounds that may be added to the vasodilator formulation.

The formulation may then be transferred to a suitable application device such as for example a pump device, which allows for an easy, no mess application.

### Example 4

A topical vasodilator formulation was prepared comprising 2.5% Arginine Hcl, 0.25% Black Pepper Essential oil and 0.25% Peppermint essential oil as per the following formulation (Table 4) to provide a formulation suitable to be used for example in a pump device.

The formulation may be used in the treatment of a disease, disorder or condition associated with a decrease and/or impairment of blood flow in a subject, in particular to treat hair loss and/or hair regrowth.

Approximately, 5% Cyanotis vaga extract supplying 50% 20-Hydroxyecdysterone and 2,5% arginine Hcl was micronized to form a powder and combined with the wetting agent ethanol. The base was formed through heating the oil phase in a container to 60 degrees and then heating the water phase in a separate container to 60 degrees. Both the oil and water phase were then combined to form an emulsion. The micronized powders of 5% Cyanotis vaga extract supplying 50% 20-Hydroxyecdysterone and 2.5% arginine Hcl to the base using a geometric dilution were combined. Black pepper and peppermint essential oil were combined with the mixture in an ointment mill to form an active blend.

Optionally, the formulation may further comprise Saw Palmetto extract - 5-alpha reductase inhibitors 2.5%, *Eclipta alba 2,5%, Crimm asiaticum* 2.5%, *Polypodium leucotomas* 2.5%, L-ascorhate 2-phosphate - 5% (or 1-ascorbate, ascorbic acid, ascorbic acid-phosphate, or any other form of ascorbates salt), niacinamide 5% nicotinic acid, methyl nicotinate" *Sea buckthorn* oil or omega 7 oil may be added to the vasodilator formulation.

The formulation may then be transferred to a suitable application device such as for example a pump device, which allows for an easy, no mess application.

### Example 5

A topical vasodilator formulation was prepared comprising 2.5% Arginine Hcl, 0.25% Black Pepper Essential oil and 0.25% Peppermint essential oil as per the following formulation (Table 5) to provide a formulation suitable to be used for example in a pump device.

The formulation may be used in the treatment of a disease, disorder or condition associated with a decrease and/or impairment of blood flow in a subject, in particular to treat aging.

Approximately, 5% Cyanotis vaga extract supplying 50% 20-Hydroxyecdysterone and 2.5% arginine Hcl was micronized to form a powder and combined with the wetting agent ethanol. The base was formed through heating the oil phase in a container to 60 degrees and then beating the water phase in a separate container to 60 degrees. Both the oil and water phase were then combined to form an emulsion. The micronized powders of 5% Cyanotis vaga extract supplying 50% 20-Hydroxytodysterone and 2.5% arginine Hcl to the base using a geometric dilution were combined Black pepper and peppermint essential oil were combined with the mixture in an ointment mill to form an active blend.

Optionally, the formulation may further comprise Bioidentical hormones including for example: testosterone, progesterone, estradiol, estrone, estriol, pregnenolone, human chorionic gonadotropin (hCG), Dehydroepiandrosterone (DHEA) and peptides GHRP-6, CJC1295, AOD9604 or derivatives thereof,

The formulation may then be transferred to a suitable application device such as for example a pump device, which allows for an easy, no mess application.

### Example 6

An alternative embodiment of a method of formulation is provided in this Example, It is envisaged that method according to the embodiment may provide a particularly stable and more effective formulation at a cheaper cost By way of Example, the method may be performed generally with reference to the components set forth in Table 6.

The method includes the following steps:
1. Add water phase ingredients to emulsifying mixer drum and mix until dissolved
2. Dissolve Cyanotis extract in ethanol in separate container
3. In a separate container heat stearic acid to make liquid and add vitamin E to liquid stearic acid
4. add step 3 to step 1 in emulsifying mixer
5. add emulsifiers to mixer and blend until smooth with no lumps
6, Add step 2 to step 5 and blend until consistent color and smooth texture
7. combine preservatives and essential oils in a separate container
7. add step 7 and step 6 in emulsifying mixer
8, Continue to mix until blended

The cyanotis extract and arginine HCl are micronized prior to their inclusion as previously described. Combine water phase ingredients demineratised water, Arginine Hcl, Aloe Barbadensis Leaf Juice, EDTA, Sorbitol solution, glycerine liquid BP/USP and Propylene Glycol into drum and blend until dissolved. In a separate container dissolve cyanotis extract in ethanol. In a separate container heat stearic acid to make liquid and then add the vitamin E liquid. Combine the liquid stearic acid and vitamin E mix into the water phase ingredients whilst mixing in the emulsifying mixer, Add the emulsifying ointment and continue to mix until lump free. Add the dissolved cyanotis extract and ethanol to the emulsifying mixing drum and continue to blend until smooth and consistent color. Combine the remaining essential oils and preservative ingredients in a separate container, Add the essential oils and preservatives to the emulsifying mixing drum and continue to blend until smooth and consistent.

The formulation may then be transferred to a suitable application device such as for example a pump device, which allows for an easy, no mess application.

Table 7 provides a specific embodiment of a rosemary oil-containing formulation that may be produced according to the method described in this Example. The method preserves the potency of the essential oils, such as on rosemary-containing formulation but also for other formulations disclosed herein Preserving the essential oil potency is assisted by avoiding the exposure to heat by only heating the stearic acid to make it liquid and combining all other ingredients at room temperature will improve the efficacy of the formulation.

Throughout the specification the aim has been to describe the preferred embodiments of the invention without limiting the invention to any one embodiment or specific collection of features. It will therefore be appreciated by those of skill in the art that, in light of the instant disclosure, various modifications and changes can be made in the particular embodiments exemplified without departing from the scope of the present invention.

**Table 1**

| Application | Body Building |
|---|---|
| Ingredients | Arginine Hcl 2.5% (0.1% to 75%) |
| | |
| | Ethanol (to wet dry ingredients) |
| | Aqua |
| | Natural Emulsifying wax (Cetearyl alcohol and Ceteareth-20) |
| | Ethyl-alcohol, Sorbitol |
| | Aloe Barbadensis Leaf Juice |
| | Stearic acid |
| | Cyclopentasiloxane |
| | Tocopheryl acetate (Vitamin E) |
| | Black Pepper Essential oil |
| | Peppermint essential oil |
| | Phenoxyethanol |
| | Caprylyl Glycol |
| Optional Ingredients | Cyanotis vaga extract or components or derivatives thereof |
| | Phytoecdysterones standardised to 50% |
| | 2O-Hydroxyecdysterone at 5% (1% to 10%) or alternative ecdysteroids |
| Functional Properties of each formulation | Vasodilation |
| | Increased protein synthesis |
| | Anti-catabolic |

**Table 2**

| Application | Peripheral Circulation (diabetes) |
|---|---|
| Ingredients | Arginine Hcl 2.5% (0.1% to 75%) |
| | |
| | Ethanol (to wet dry ingredients) |
| | Aqua |
| | Natural Emulsifying wax (Cetearyl alcohol and Ceteareth-20) |
| | Ethyl-alcohol, Sorbitol |
| | Aloe Barbadensis Leaf Juice |
| | Stearic acid |
| | Cyclopentasiloxane |
| | Tocopheryl acetate (Vitamin E) |
| | Black Pepper Essential oil |
| | Peppermint essential oil |
| | Phenoxyethanol |
| | Caprylyl Glycol |
| Optional Ingredients | Antimicrobial antiseptic(s): Kunzea ambigua oil, Tea tree oil |
| | Cyanotis vaga extract standardised to 50% 20-Hydroxyecdysterone at 5% (1% to 10%) or alternative ecdysteroids |
| Functional Properties of each formulation | Vasodilation |
| | Increased wound healing |
| | Lower blood glucose |

**Table 3**

| Application | Erectile Dysfunction and female sexual dysfunction |
|---|---|
| Ingredients | Arginine Hcl 2,5% (0.1% to 75%) |
| | |
| | Ethanol (to wet dry ingredients) |
| | Aqua |
| | Natural Emulsifying wax (Cetearyl alcohol and Ceteareth-20) |
| | Ethyl-alcohol, Sorbitol |
| | Aloe Barbadensis Leaf Juice |
| | Stearic acid |
| | Cyclopentasiloxane |
| | Tocopheryl acetate (Vitamin E) |
| | Black Pepper Essential oil |
| | Peppermint essential oil |
| | Phenoxyethanol |
| | Caprylyl Glycol |
| Optional Ingredients | PDE inhibitors - Quercetin |
| | Alpha-2 adrenoceptor antagonists - Yohimbine Hcl |
| | Lysine - reduce risk of arginine flare of herpes virus in predisposed/infected individuals |
| | Alternative base can be used to work as a "lubricant" |
| Functional Properties of each formulation | Vasodilation |
| | PDE4 and PDE5 inhibition |
| | Reduce risk of HPV flare and subsequent spread |
| | Topical cream and/or lubricant to enhance sexual performance in males and females |

**Table 4**

| Application | Hair Loss / hair regrowth |
|---|---|
| Ingredients | Arginine Hcl 2.5% (0.1% to 75%) |
| | |
| | Ethanol (to wet dry ingredients) |
| | Aqua |
| | Natural Emulsifying wax (Cetearyl alcohol and Ceteareth-20) |
| | Ethyl-alcohol, Sorbitol |
| | Aloe Barbadensis Leaf Juice |
| | Stearic acid |
| | Cyclopentasiloxane |
| | Tocopheryl acetate (Vitamin E) |
| | Black Pepper Essential oil |
| | Peppermint essential oil |
| | Phenoxyethanol |
| | Caprylyl Glycol |
| Optional Ingredients | Saw Palmetto extract - 5 alpha reductase inhibitors |
| | 2.5% (0.5% to 25%) |
| | Eclipta alba 2.5% - (0.5% to 25%) |
| | Crinum asiaticum 2.5% - (0.5% to 25%) |
| | Polypodium leucotomas 2.5% - (0.5% to 25%) |
| | L-ascorbate 2-phosphate - 5% (or 1-ascorbate, ascorbic acid, ascorbic acid-2-phosphate, or any other form of ascorbatss salt) (0.1% to 20%) |
| | niacinamide 5% nicotinic acid, methyl nicotinate (0.1% to 10%) |
| | Sea buckthorn oil - omega 7 oil (0.5% to 10%) |
| Functional Properties of each formulation | Vasodilation |
| | Reduce hair loss |
| | chance Hair regrowth |
| | Improve health of scalp |
| | Suitable for male and female pattern baldness and all |
| | forms of alopecia |

**Table 5**

| Application | Anti-aging |
|---|---|
| Ingredients | Arginine Hcl 2.5% (0.1% to 75%) |
| | |
| | Ethanol (to wet dry ingredients) |
| | Aqua |
| | Natural Emulsifying wax (Cetearyl alcohol and Ceteareth-20) |
| | Ethyl-alcohol, Sorbitol |
| | Aloe Barbadensis Leaf Juice |
| | Stearic acid |
| | Cyclopentasiloxane |
| | Tocopheryl acetate (Vitamin E) |
| | Black Pepper Essential oil |
| | Peppermint essential oil |
| | Phenoxyethanol |
| | Caprylyl Glycol |
| Optional Ingredients | Bioidentical hormones |
| Functional Properties of each formulation | Vasodilator and penetration enhances to increase the absorption and utilization of bio-identical hormones |

**Table 6**

| **water phase** |
|---|
| Demineralised water |
| Arginine Hcl (2.5%) |
| Aloe Barbadensis Leaf Juice |
| EDTA |
| Sorbitol solution |
| glycerine liquid BP/USP |
| Propylene Glycol |

| **oil phase** |
|---|
| Stearic acid |
| Tocopheryl acetate (Vitamin E) liquid BP/USP |

| **emulsifiers** |
|---|
| Emulsifying Wax |
| White Soft Paraffin |
| Liquid Paraffin |

| **preservatives and essential oils** |
|---|
| dimethicone 100cs |
| Cyclomethicone |
| Rosemary essential oil |
| Black Pepper Essential oil (0.25%) |
| Pepperment essential oil (0.1%) |
| phenoxyethanol |
| caprylyl glycol |

**Table 7**

| | | |
|---|---|---|
| Cyanotis vaga extract (50% 20-Hydroxyecdysterone) | 3g | |
| Arginine Hcl | 1.5g | |
| Black Pepper Essential oil | 0.15ml | |
| Peppermint essential oil | 0.06ml | |
| Rosemary essential oil | 5ml | |
| Demineralised water | 32.6ml | |
| Emulsifying Wax | 5g | |
| White Soft Paraffin | 8.45g | |
| Liquid Paraffin | 3.3ml | |
| Ethyl-alcohol | 6.6ml | |
| Sorbitol solution | 1.36ml | |
| Aloe Barbadensis Leaf Juice | 1.36ml | |
| Stearic acid | 1.36g | |
| Cyclomethicone | 0.27ml | |
| dimethicone 100cs | 0.27ml | |
| Tocopheryl acetate (Vitamin E) liquid BP/USP | 0.27ml | |
| glycerine liquid BP/USP | 10.86ml | |
| Propylene Glycol | 1.35ml | |
| phenoxyethanol | 0.396ml | |
| caprylyl glycol | 0.264ml | |
| EDTA | 0.014g | |

## Claims

1. A topically administrable vasodilator formulation comprising:
(i) arginine;
(ii) black pepper extract; and
(iii) peppermint extract.

2. The vasodilator formulation of claim 1, wherein the formulation comprises from about 0.1% to about 75% of arginine, or preferably about 2.5% arginine.

3. The vasodilator formulation of claim 1 or claim 2, wherein the formulation comprises from about 0.01% to about 25% black pepper extract, or preferably about 0.25% black pepper extract.

4. The vasodilator formulation of any one of claims 1 to 3, wherein the formulation comprises from about 0.01% to about 25% peppermint extract, or preferably about 0.25% peppermint extract.

5. The vasodilator formulation of any one of claims 1 to 4, wherein the formulation further comprises rosemary oil.

6. The vasodilator formulation of any one of claims 1 to 5, wherein the formulation further comprises one or more penetration enhancers, preferably wherein the penetration enhancer is Aloe vera extract and/or stearic acid.

7. The vasodilator formulation of any one of claims 1 to 6, further comprising at least one pharmaceutically acceptable carrier, diluent and/or excipient, preferably an oil-in-water emulsion.

8. The vasodilator formulation of any one of claims 1 to 7, further comprising at least one terpene.

9. The vasodilator formulation of any one of claims 1 to 8, further comprising an ecdysteroid; an antimicrobial; an alpha-2 antagonist; a phosphodiesterase-5a inhibitor; lysine; a 5-alpha reductase inhibitor; a hair growth promoter; at least one immune and/or cytokine modulator; an additional local vasodilator; and/or a Hippophae rhamnoide extract.

10. The vasodilator formulation of any one of claims 1 to 9, wherein the formulation is an oil, mousse, gel, cream, lotion, balm, lubricant, foam, liquid or aerosol, self-tanning lotion, oil, spray or paint.

11. The vasodilator formulation of any one of claims 1 to 10, wherein arginine is selected from the group consisting of: 2-Amino-5-guanidinopentanoic acid, agmatine, arginine hydrochloride, Ark 1, decarboxylated arginine, dipeptide arginyl aspartate, D-arginine, L-arg, L-arginine, L-arginine aspartate, NG-monomethyl-L-arginine, arginine alpha ketogluterate, arginine ethyl esther, norvaline, arginine salt, arginine ester, Argivene, Detoxargin, Levargin, Minophagen Argamine, Polyarginine, Arginina, (S)-2-Amino-5-guanidinopentanoic acid, 2-amino-5-guanidinovaleric acid, Argininum [INN-Latin], Arginina [INN-Spanish], L-alpha-Amino-delta-guanidinovaleric acid, L-Arginin, Poly(L-arginine), L-Ornithine, N5-(aminoiminomethyl)-, L(+)-Arginine, 1-Amino-4-guanidovaleric acid, H-Arg-OH, L-a-Amino-d-guanidinovaleric acid, L-Arginine, homopolymer, (S)-2-Amino-5-[(aminoiminomethyl)amino]pentanoic acid, L-Arginine hydrochloride, L-Norvaline, 5-((aminoiminomethyl)amino)-, (S)-2-Amino-5-guanidinovaleric acid, (2S)-2-amino-5-(diaminomethylideneamino)pentanoic acid, (S)-2-Amino-5-((aminoiminomethyl)amino)pentanoic acid, Pentanoic acid, 2-amino-5-((aminoiminomethyl)amino, L-Norvaline, 5-[(aminoiminomethyl)amino]- 2-Amino-5-Guanidnovaleric Acid, Argininum Pentanoic acid, 2-amino-5-[(aminoiminomethyl)amino]-, Tocris-0663, L-Arginine (JP16), Lopac-A-5006, Arginine, 2-amino-5-guanidinovalerate, Arginine hydrochloride (USAN), L-a-Amino-d-guanidinovalerate, N5-(aminoiminomethyl)-L-Ornithine, L-alpha-Amino-delta-guanidinovalerate, (2S)-2-amino-5-guanidinopentanoic acid, 5-[(aminoiminomethyl)amino]-L-Norvaline, (2S)-2-amino-5-(carbamimidamido)pentanoic acid, L-Arginine-L-Glutamate 2-Amino-Pentanedoic Acid, (S)-2-amino-5-[(aminoiminomethyl)amino]-Pentanoate, (S)-2-amino-5-[(aminoiminomethyl)amino]-Pentanoic acid, (2S)-2-azanyl-5-[bis(azanyl)methylideneamino]pentanoic acid, alpha-keto-gamma-guanidovaleric acid, ornithine, citrulline, guanidoacetic acid and ornithine.

12. A method of producing the topically administrable vasodilator formulation according to any one of claims 1 to 11, including the step of. combining arginine; black pepper extract; and peppermint extract to thereby produce the topically administrable vasodilator formulation.

13. A topically administrable vasodilator formulation according to any one of claims 1 to 11, or produced according to the method of claim 12 for use in the therapeutic and/or prophylactic treatment of a disease, disorder or condition associated with a decrease and/or impairment of blood flow and/or vasodilation in a subject, wherein the disease, disorder or condition associated with a decrease and/or impairment of blood flow is selected from the group consisting of: erectile dysfunction, female sexual dysfunction, male sexual dysfunction and diabetes.

14. A non-medical method of enhancing muscular and/or vascular definition in a subject, the method including the step of topically administering to said subject an effective amount of a topical vasodilator formulation according to any one of claims 1 to 11, or produced according to the method of claim 12, to enhance muscular and/or vascular definition in the subject.

15. A kit comprising the vasodilator formulation according to any one of claims 1 to 11 or produced according to the method of claim 12, an applicator device and instructions for using said formulation to increase, enhance and/or stimulate blood flow and/or vasodilation in a subject.

## Patentansprüche

1. Topisch verabreichbare Vasodilatatorformulierung, umfassend:
(i) Arginin;
(ii) Extrakt aus schwarzem Pfeffer; und
(iii) Pfefferminz-Extrakt.

2. Vasodilatatorformulierung nach Anspruch 1, wobei die Formulierung etwa 0,1% bis etwa 75% Arginin oder vorzugsweise etwa 2,5% Arginin umfasst.

3. Vasodilatatorformulierung nach Anspruch 1 oder Anspruch 2, wobei die Formulierung etwa 0,01% bis etwa 25% Extrakt aus schwarzem Pfeffer oder vorzugsweise etwa 0,25% Extrakt aus schwarzem Pfeffer umfasst.

4. Vasodilatatorformulierung nach einem der Ansprüche 1 bis 3, wobei die Formulierung etwa 0,01% bis etwa 25% Pfefferminz-Extrakt oder vorzugsweise etwa 0,25% Pfefferminz-Extrakt umfasst.

5. Vasodilatatorformulierung nach einem der Ansprüche 1 bis 4, wobei die Formulierung ferner Rosmarinöl umfasst.

6. Vasodilatatorformulierung nach einem der Ansprüche 1 bis 5, wobei die Formulierung ferner einen oder mehrere Penetrationsverstärker umfasst, wobei vorzugsweise der Penetrationsverstärker Aloe-Vera-Extrakt und/oder Stearinsäure ist.

7. Vasodilatatorformulierung nach einem der Ansprüche 1 bis 6, ferner umfassend mindestens einen pharmazeutisch verträglichen Träger, Verdünner und/oder Hilfsstoff, vorzugsweise eine Öl-in-Wasser-Emulsion.

8. Vasodilatatorformulierung nach einem der Ansprüche 1 bis 7, ferner umfassend mindestens ein Terpen.

9. Vasodilatatorformulierung nach einem der Ansprüche 1 bis 8, ferner umfassend ein Ecdysteroid; ein antimikrobielles Mittel; ein alpha-2-Antagonist; ein Phosphodiesterase-5a-Inhibitor; Lysin; ein 5-alpha-Reduktase-Inhibitor; ein Haarwuchsförderer; mindestens ein Immun- und/oder Zytokinmodulator; einen zusätzlichen lokalen Vasodilatator; und/oder ein Hippophae-Rhamnoid-Extrakt.

10. Vasodilatatorformulierung nach einem der Ansprüche 1 bis 9, wobei die Formulierung ein Öl, ein Mousse, ein Gel, eine Creme, eine Lotion, ein Balsam, ein Gleitmittel, ein Schaum, eine Flüssigkeit oder ein Aerosol, eine Selbstbräunungslotion, ein Öl, ein Spray oder eine Tinktur ist.

11. Vasodilatatorformulierung nach einem der Ansprüche 1 bis 10, wobei Arginin ausgewählt ist aus der Gruppe bestehend aus: 2-Amino-5-guanidinopentansäure, Agmatin, Argininhydrochlorid, Ark 1, decarboxyliertem Arginin, Dipeptid Arginylaspartat, D-Arginin, L-Arg, L-Arginin, L-Argininaspartat, NG-Monomethyl-L-Arginin, Arginin-alpha-Ketogluterat, Argininethylester, Norvalin, Argininsalz, Argininester, Argivene, Detoxargin, Levargin, Minophagen-Argamin, Polyarginin, Arginina, (S)-2-Amino-5-guanidinopentansäure, 2-Amino-5-guanidinovaleriansäure, Argininum [INN-Latin], Arginina [INN-Spanish], L-alpha-Amino-delta-guanidinovaleriansäure, L-Arginin, Poly(L-Arginin), L-Ornithin, N5-(Aminoiminomethyl)-, L(+)-Arginin, 1-Amino-4-guanidovaleriansäure, H-Arg-OH, L-a-Amino-d-guanidinovaleriansäure, L-Arginin, Homopolymer, (S)-2-Amino-5-[(aminoiminomethyl)amino]pentansäure, L-Argininhydrochlorid, L-Norvalin, 5-((Aminoiminomethyl)amino)-, (S)-2-Amino-5-guanidinovaleriansäure, (2S)-2-Amino-5-(diaminomethylidenamino)pentansäure, (S)-2-Amino-5-((aminoiminomethyl)amino)pentansäure, Pentansäure, 2-Amino-5-((aminoiminomethyl)amino, L-Norvalin, 5-[(Aminoiminomethyl)amino]-2-amino-5-guanidinovaleriansäure, Argininum-pentansäure, 2-Amino-5-[(aminoiminomethyl)amino]-, Tocris-0663, L-Arginin (JP16), Lopac-A-5006, Arginin, 2-Amino-5-guanidinovalerat, Argininhydrochlorid (USAN), L-a-Amino-d-guanidinovalerat, N5-(Aminoiminomethyl)-L-Ornithin, L-alpha-Amino-delta-guanidinovalerat, (2S)-2-Amino-5-guanidinopentansäure, 5-[(Aminoiminomethyl)amino]-L-Norvalin, (2S)-2-Amino-5-(carbamimidamido)pentansäure, L-Arginin-L-Glutamat-2-aminopentandisäure, (S)-2-Amino-5-[(aminoiminomethyl)amino]pentanoat, (S)-2-Amino-5-[(Aminoiminomethyl)amino]pentansäure, (2S)-2-Azanyl-5-[bis(azanyl)methylidenamino]pentansäure, alpha-Keto-gamma-Guanidovaleriansäure, Ornithin, Citrullin, Guanidoessigsäure und Ornithin.

12. Verfahren zur Herstellung der topisch verabreichbaren Vasodilatatorformulierung nach einem der Ansprüche 1 bis 11, umfassend den Schritt von Kombinieren von Arginin; Extrakt aus schwarzem Pfeffer; und Pfefferminz-Extrakt, um dadurch die topisch verabreichbare Vasodilatatorformulierung herzustellen.

13. Topisch verabreichbare Vasodilatatorformulierung nach einem der Ansprüche 1 bis 11 oder hergestellt nach dem Verfahren von Anspruch 12 zur Verwendung bei der therapeutischen und/oder prophylaktischen Behandlung einer Krankheit, Störung oder eines Zustands, die mit einer Abnahme und/oder Beeinträchtigung des Blutflusses und/oder Vasodilatation in einem Subjekt verbunden ist, wobei die Krankheit, Störung oder der Zustand, die mit einer Abnahme und/oder Beeinträchtigung des Blutflusses verbunden sind, ausgewählt sind aus der Gruppe bestehend aus: erektiler Dysfunktion, weiblicher sexueller Dysfunktion, männlicher sexueller Dysfunktion und Diabetes.

14. Nichtmedizinisches Verfahren zur Verbesserung der muskulären und/oder vaskulären Definition in einem Subjekt, wobei das Verfahren den Schritt einer topischen Verabreichung einer wirksamen Menge einer topischen Vasodilatatorformulierung nach einem der Ansprüche 1 bis 11 oder hergestellt nach dem Verfahren von Anspruch 12 an das Subjekt umfasst, um die muskuläre und/oder vaskuläre Definition in dem Subjekt zu verbessern.

15. Kit, umfassend die Vasodilatatorformulierung nach einem der Ansprüche 1 bis 11 oder hergestellt nach dem Verfahren von Anspruch 12, eine Applikatorvorrichtung und Anweisungen zur Verwendung der Formulierung zur Erhöhung, Verbesserung und/oder Stimulierung des Blutflusses und/oder der Vasodilatation in einem Subjekt.

## Revendications

1. Formulation vasodilatatrice administrable par voie topique comprenant:
(i) de l'arginine;
(ii) un extrait de poivre noir; et
(iii) un extrait de menthe poivrée.

2. Formulation vasodilatatrice selon la revendication 1, dans laquelle la formulation comprend d'environ 0,1% à environ 75% d'arginine, ou de préférence environ 2,5% d'arginine.

3. Formulation vasodilatatrice selon la revendication 1 ou la revendication 2, dans laquelle la formulation comprend d'environ 0,01 % à environ 25 % d'extrait de poivre noir, ou de préférence environ 0,25 % d'extrait de poivre noir.

4. Formulation vasodilatatrice selon l'une quelconque des revendications 1 à 3, dans laquelle la formulation comprend d'environ 0,01 % à environ 25 % d'extrait de menthe poivrée, ou de préférence environ 0,25 % d'extrait de menthe poivrée.

5. Formulation vasodilatatrice selon l'une quelconque des revendications 1 à 4, dans laquelle la formulation comprend en outre de l'huile de romarin.

6. Formulation vasodilatatrice selon l'une quelconque des revendications 1 à 5, dans laquelle la formulation comprend en outre un ou plusieurs activateurs de pénétration, de préférence dans laquelle l'activateur de pénétration est un extrait d'aloé véra et/ou de l'acide stéarique.

7. Formulation vasodilatatrice selon l'une quelconque des revendications 1 à 6, comprenant en outre au moins un véhicule, diluant et/ou excipient pharmaceutiquement acceptable, de préférence une émulsion huile dans l'eau.

8. Formulation vasodilatatrice selon l'une quelconque des revendications 1 à 7, comprenant en outre au moins un terpène.

9. Formulation vasodilatatrice selon l'une quelconque des revendications 1 à 8, comprenant en outre un ecdystéroïde; un antimicrobien; un antagoniste alpha-2; un inhibiteur de phosphodiestérase 5a; de la lysine; un inhibiteur de 5-alpha réductase; un promoteur de croissance des cheveux; au moins un modulateur immun et/ou cytokine; un vasodilatateur local supplémentaire et/ou un extrait de rhamnoïde Hippophae.

10. Formulation vasodilatatrice selon l'une quelconque des revendications 1 à 9, dans laquelle la formulation est une huile, une mousse, un gel, une crème, une lotion, un baume, un lubrifiant, un produit moussant, un liquide ou un aérosol, une lotion autobronzante, une huile, un spray ou une peinture.

11. Formulation vasodilatatrice selon l'une quelconque des revendications 1 à 10, dans laquelle l'arginine est choisie parmi le groupe comprenant: acide 2-amino-5-guanidinopentanoïque, agmatine, chlorhydrate d'arginine, l'Ark 1, arginine décarboxylée, dipeptide arginyl aspartate, D-arginine, L-arg, L-arginine, L-arginine aspartate, NG-méthyl-L-arginine, arginine alpha cétogluterate, éther éthylique d'arginine, norvaline, sel d'arginine, ester d'arginine, argivène, détoxargine, lévargine, argamine minophagène, polyarginine, arginine, acide (S)-2-amino-5-5-0 guanidinopentanoïque, acide 2-amino-5-guanidinovalérique, argininum[INN-Latin], arginina[INN-Spanish], acide L-alpha-amino-delta-guanidinovalérique, L-arginine, Poly(L-arginine), L-ornithine, N5-(aminoiminométhyle)-, L(+)-arginine, acide 1-amino-4-guanidovalérique, H-Arg-OH, acide L-a-amino-d-guanidinovalérique, L-arginine, homopolymère, acide (S)-2-amino-5-[(aminoiminométhyl)amino]pentanoic, chlorhydrate L-arginine, L-norvaline, acide 5-((aminoiminométhyl)amino)-, (S)-2-Amino-5-guanidinovalérique, acide (2S)-2-amino-5-(diaminométhylidèneamino)pentanoïque, acide (S)-2-Amino-5-((aminoiminométhyl) amino) pentanoïque, acide penstanoïque, 2-amino-5-((aminoiminométhyl)amino, L-norvaline, acide 5-[(aminoiminométhyl)amino]-2-amino-5-guanidnovalérique, acide d'argininum pentanoïque, 2-amino-5-[(aminoiminométhyl)amino]-, Tocris-0663, L-arginine (JP16), Lopac-A-5006, Arginine, 2-amino-5-guanidinovalérate, chlorhydrate d'arginine (USAN), L-a-Amino-d-guanidino valérate, N5-(aminoiminométhyl)-L-ornithine, L-alpha-Amino-delta-guanidinovalérate, acide (2S)-2-amino-5-guanidinopentanoïque, 5-[(aminoiminométhyl)amino]-L-norvaline, acide (2S)-2-amino-5-(carbamimidamido) pentanoïque, acide lopadesrginine-L-glutamate 2-amino pentanoïque, (S)-2-amino-5-[(aminoiminométhyl)amino]-pentanoate, acide (S)-2-amino-5-[(aminoiminométhyl)amino] pentanoïque, acide (2S)-2-azanyl-2-azanyl-5-[bis(azanyl)méthylidèneamino]pentanoïque, acide alpha-cétogamma-guanidovalérique, ornithine, citrulline, acide guanidacétique et ornithine.

12. Procédé de production de la formulation vasodilatatrice administrable par voie topique selon l'une quelconque des revendications 1 à 11, comprenant l'étape consistant à combiner de l'arginine; un extrait de poivre noir et un extrait de menthe poivrée pour produire ainsi la formulation vasodilatatrice administrable par voie topique.

13. Formulation vasodilatatrice administrable par voie topique selon l'une quelconque des revendications 1 à 11, ou produite selon le procédé selon la revendication 12 pour une utilisation dans le traitement thérapeutique et/ou prophylactique d'une maladie, d'un trouble ou d'un état associé à une diminution et/ou une altération du débit sanguin et/ou une vasodilatation chez un sujet, la maladie, le trouble ou l'état associé à une diminution et/ou une altération du débit sanguin parmi: un dysfonctionnement érectile, un dysfonctionnement sexuel féminin, un dysfonctionnement sexuel masculin et le diabète.

14. Procédé non médical d'amélioration de la définition musculaire et/ou vasculaire chez un sujet, le procédé comprenant l'étape d'administration topique audit sujet d'une quantité efficace d'une formulation vasodilatatrice topique selon l'une quelconque des revendications 1 à 11, ou produite selon le procédé selon la revendication 12, pour améliorer la définition musculaire et/ou vasculaire chez ce sujet.

15. Kit comprenant la formulation vasodilatatrice selon l'une quelconque des revendications 1 à 11 ou produite selon le procédé de la revendication 12, un dispositif applicateur et des instructions pour utiliser ladite formulation pour augmenter, améliorer et/ou stimuler la circulation sanguine et/ou la vasodilatation chez un sujet.
